# EUROPEAN PATENT SPECIFICATION

(11) **EP 4 048 338 B1**
(45) Date of publication and mention of the grant of the patent: **19.02.2025**
(21) Application number: 20761475.1
(22) Date of filing: 13.08.2020
(51) Int. Cl.: A61M 1/16, A61M 1/28, G16H 40/40

(54) **MAINTENANCE NOTIFICATION FOR MEDICAL DEVICES**
WARTUNGSBENACHRICHTIGUNG FÜR MEDIZINISCHE VORRICHTUNGEN
NOTIFICATION DE MAINTENANCE POUR DISPOSITIFS MÉDICAUX

(30) Priority: 25.10.2019 US 201916663656
(43) Date of publication of application: 31.08.2022
(73) Proprietor: Fresenius Medical Care Holdings, Inc., Waltham, MA 02451 (US)
(72) Inventor: SCHMIDT, Daniel H., Petaluma, CA 94954 (US); EGLEY, Bert D., Walnut Creek, CA 94598 (US)
(74) Representative: Freischem & Partner Patentanwälte mbB
(86) International application number: PCT/US2020/046098
(87) International publication number: WO 2021/080667

(56) References cited:
- US-A1- 2014 266 713
- US-A1- 2018 093 027
- US-B2- 7 873 489

## Description

### TECHNICAL FIELD

This application relates to medical systems and more particularly to medical dialysis systems that determine when different components thereof need maintenance.

### BACKGROUND

Dialysis machines are known for use in the treatment of renal disease. Two types of dialysis methods are hemodialysis (HD) and peritoneal dialysis (PD). During hemodialysis, the patient's blood is passed through a dialyzer of a hemodialysis machine while also passing dialysate through the dialyzer. A semi-permeable membrane in the dialyzer separates the blood from the dialysate within the dialyzer and allows diffusion and osmosis exchanges to take place between the dialysate and the blood stream. During peritoneal dialysis, the patient's peritoneal cavity is periodically infused with dialysate or dialysis solution. The membranous lining of the patient's peritoneum acts as a natural semi-permeable membrane that allows diffusion and osmosis exchanges to take place between the solution and the blood stream. Automated peritoneal dialysis machines, also called PD cyclers, are designed to control the entire peritoneal dialysis process so that it can be performed at home, usually overnight, without clinical staff in attendance.

Both peritoneal dialysis and hemodialysis machines include several electromechanical subsystems that need to work reliably over the useful life of the product. In some cases, components of electromechanical subsystems are replaced or maintained as part of routine preventive maintenance procedures. Due to the diverse nature of treatment options, not all equipment components are used in a uniform or completely predictable manner in between maintenance intervals. Furthermore, dialysis machines can operate in multiple modes that use different electromechanical subsystems differently depending on the mode. Current technologies to monitor system use, such as an overall system power-on hour meter, do not fully account for the fact that different subsystems may be used more or less heavily than the same subsystem in a different piece of dialysis equipment over the same maintenance interval. Accordingly, some components may be replaced before there is any wear and thus before the components need to be replaced while other components may have already exceeded their useful life by the time they are scheduled to be replaced.

It is referred to US 7,873,489 B2, US 2014/0266713 A1 and US 2018/0093027 A1 as prior art.

Accordingly, it is desirable to determine when to replace dialysis machine components only after the components are sufficiently worn but before the components have exceeded their useful life.

### SUMMARY

A medical device according to the present invention comprises the technical features as defined in independent claim 1. A method of determining when maintenance needs to be performed for electromechanical components of a medical device according to the present invention comprises the technical features as defined in independent claim 5. A non-transitory computer readable medium containing software that determines when maintenance needs to be performed for electromechanical components of a medical device according to the present invention comprises the technical features as defined in independent claim 9. A dialysis system according to the present invention comprises the technical features as defined in independent claim 13.

According to the system described herein, a medical device includes a plurality of electromechanical components, and a control unit, coupled to the electromechanical components, that actuates the electromechanical components and, for each of the electromechanical components, maintains a count of a number of times a respective one of the electromechanical components is actuated and/or a run time duration of the respective one of the electromechanical components. The medical device also includes a table having observed values for each of the electromechanical components, where a maintenance alert is provided for at least one of the electromechanical components in response to the count and/or the run time for the at least one of the electromechanical components exceeding a corresponding maintenance limit for the electromechanical component and where the maintenance limits are independent for different ones of the electromechanical components. The medical device may be a dialysis machine. At least some of the maintenance limits may be determined by analyzing prior maintenance information. The medical device includes a plurality of sensors, coupled to the control unit, that measure a plurality of secondary parameters that cause a maintenance alert to be provided. The table maintains values for the secondary parameters. One of the secondary parameters is temperature. The electromechanical components may include pumps, valves, actuators, sensors, gears, motors, and/or solenoids.

According further to the system described herein, determining when maintenance needs to be performed for electromechanical components of a medical device includes maintaining in a table a count of a number of times a respective one of the electromechanical components is actuated and/or a run time duration of the respective one of the electromechanical components in connection with actuating each of the electromechanical components and includes providing a maintenance alert for at least one of the electromechanical components in response to the count and/or the run time for the at least one of the electromechanical components exceeding a corresponding maintenance limit for the electromechanical component, where the maintenance limits are independent for different ones of the electromechanical components. The medical device may be a dialysis machine. At least some of the maintenance limits may be determined by analyzing prior maintenance information. The table maintains values for secondary parameters that cause a maintenance alert to be provided. One of the secondary parameters is temperature. The electromechanical components may include pumps, valves, actuators, sensors, gears, motors, and/or solenoids.

According further to the system described herein, a non-transitory computer readable medium contains software that determines when maintenance needs to be performed for electromechanical components of a medical device. The software includes executable code that maintains in a table a count of a number of times a respective one of the electromechanical components is actuated and/or a run time duration of the respective one of the electromechanical components in connection with actuating each of the electromechanical components. The software also includes executable code that provides a maintenance alert for at least one of the electromechanical components in response to the count and/or the run time for the at least one of the electromechanical components exceeding a corresponding maintenance limit for the electromechanical component, where the maintenance limits are independent for different ones of the electromechanical components. The medical device may be a dialysis machine. At least some of the maintenance limits may be determined by analyzing prior maintenance information. The table maintains values for secondary parameters that cause a maintenance alert to be provided. One of the secondary parameters is temperature. The electromechanical components may include pumps, valves, actuators, sensors, gears, motors, and/or solenoids.

According further to the system described herein, a dialysis system includes a dialysis machine. The dialysis machine includes a housing, a dialysate circuit, at least a portion of which is disposed inside the housing, the dialysate circuit circulating dialysate to remove toxins from blood of a patient coupled to the dialysis system, and a plurality of electromechanical components. The system further includes a control unit having a processor that executes software in a non-transitory computer readable medium, the software operating the dialysate circuit and including executable code that maintains in a table at least one of: a count of a number of times a respective one of the electromechanical components is actuated or a run time duration of the respective one of the electromechanical components in connection with actuating each of the electromechanical components and executable code that provides a maintenance alert for at least one of the electromechanical components in response to at least one of: the count or the run time for the at least one of the electromechanical components exceeding a corresponding maintenance limit for the electromechanical component, where the maintenance limits are independent for different ones of the electromechanical components.

### BRIEF DESCRIPTION OF THE DRAWINGS

Embodiments and features of the system described herein are explained with reference to the several figures of the drawings, which are briefly described as follows.
FIG. 1 shows a perspective view of a hemodialysis system according to an embodiment of the system described herein.
FIG. 2 shows a perspective view of a peritoneal dialysis system according to an embodiment of the system described herein.
FIG. 3 is a schematic diagram illustrating a control unit, actuators, and sensors according to an embodiment of the system described herein.
FIG. 4 shows a table containing entries for components of a system according to an embodiment of the system described herein.
FIG. 5 is a flow diagram illustrating processing performed in connection with actuating a component of a system according to an embodiment of the system described herein.
FIG. 6 is a flow diagram illustrating processing performed in connection with deactivating a component of a system according to an embodiment of the system described herein.

### DETAILED DESCRIPTION

The system described herein uses software or firmware that controls electromechanical components of a dialysis machine to count and record usage characteristics of subsystems to better inform maintenance technicians about component replacement. Specifically, in response to software sending a command to actuate an electromechanical component, a counter corresponding to that component is increment and/or a timer is started. Counters and/or total run time values for all of the monitored components are maintained in persistent memory. When a counter exceeds a predetermined level specific to the corresponding component and/or when an accumulated run time for the component exceeds a predetermined level specific to the corresponding component, a notification is sent to indicate that the component has reached or will soon reach the end of useful life of the component. The notification may be displayed to a user, to a clinician and/or to a technician using specific graphical user interface windows or the notification may be combined with other similar maintenance notifications for other components. A single notification may be displayed that includes all components requiring maintenance in an upcoming routine maintenance. It is also possible that software similar to that which is used to control electromechanical components may also be used to monitor use of strictly electrical components such as resistors, capacitors, etc. For electrical components rated to a specific useful life, current, or duty cycle the software could record when limits with respect to these ratings are reached or nearly reached. As components are replaced, counters within the system software may be reset for the components. For many electromechanical subsystems where the lifetime may be a function of the operating temperature, the monitoring system may log both the operating temperature and the usage hours, and use both in a calculation to determine if maintenance is required. In addition, historical cycle count and duration data stored in the system may be used to provide reliability data for future engineering purposes.

The system described herein may provide cost savings due to prevention of unplanned system down time and unnecessary disposal of components that have not yet reached an end-of-life state. Additionally, data from the system described herein may be used for improvement of future engineering activities related to designing for reliability.

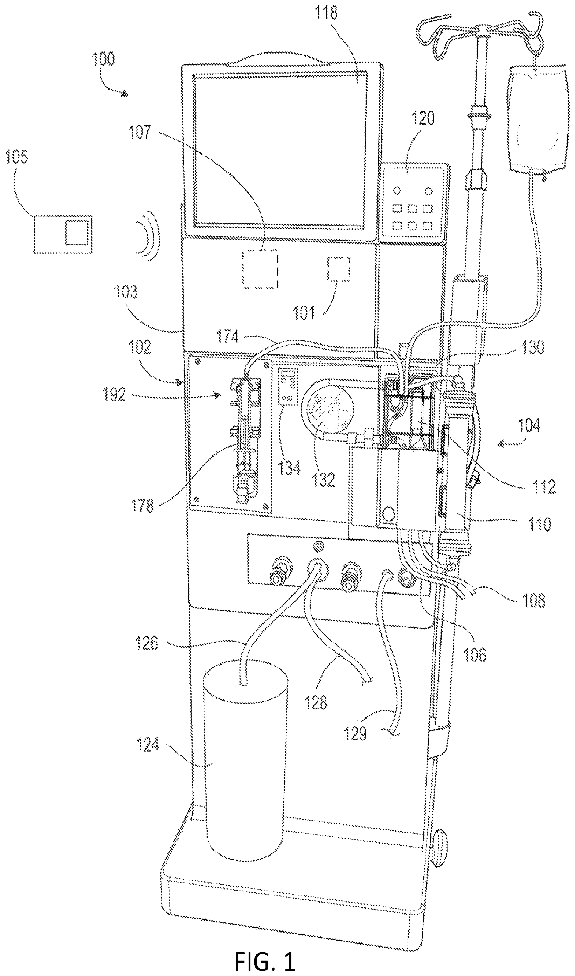

FIG. 1 shows a perspective view of a hemodialysis system 100 that includes a hemodialysis machine 102 connected to a disposable blood component set 104 that partially forms a blood circuit. During hemodialysis treatment, an operator connects arterial and venous patient lines 106, 108 of the blood component set 104 to a patient. The blood component set 104 includes an air release device 112, which contains a self-sealing vent assembly that allows air but does not allow liquid to pass. As a result, if blood passing through the blood circuit during treatment contains air, the air release device 112 will vent the air to atmosphere.

The blood component set 104 is secured to a module 130 attached to the front of the hemodialysis machine 102. The module 130 includes the blood pump 132 capable of circulating blood through the blood circuit. The module 130 also includes various other instruments capable of monitoring the blood flowing through the blood circuit. The module 130 includes a door that when closed, as shown in FIG. 1, cooperates with the front face of the module 130 to form a compartment sized and shaped to receive the blood component set 104. In the closed position, the door presses certain blood components of the blood component set 104 against corresponding instruments exposed on the front face of the module 130.

The operator uses a blood pump module 134 to operate the blood pump 132. The blood pump module 134 may include a display window, a start/stop key, an up key, a down key, a level adjust key, and an arterial pressure port. The display window displays the blood flow rate setting during blood pump operation. The start/stop key starts and stops the blood pump 132. The up and down keys increase and decrease the speed of the blood pump 132. The level adjust key raises a level of fluid in an arterial drip chamber. The controls may exist as physical buttons/switches/actuators/etc. or wholly within a graphical user interface on a touch screen accessible display 118 or some combination thereof.

The hemodialysis machine 102 further includes a dialysate circuit formed by the dialyzer 110, various other dialysate components, and dialysate lines connected to the hemodialysis machine 102. Many of these dialysate components and dialysate lines are inside the housing 103 of the hemodialysis machine 102 and are thus not visible in FIG. 1. During treatment, while the blood pump 132 circulates blood through the blood circuit, dialysate pumps (not shown) circulate dialysate through the dialysate circuit.

A dialysate container 124 is connected to the hemodialysis machine 102 via a dialysate supply line 126. A drain line 128 and an ultrafiltration line 129 also extend from the hemodialysis machine 102. The dialysate supply line 126, the drain line 128, and the ultrafiltration line 129 are fluidly connected to the various dialysate components and dialysate lines inside the housing 103 of the hemodialysis machine 102 that form part of the dialysate circuit. During hemodialysis, the dialysate supply line 126 carries fresh dialysate from the dialysate container 124 to the portion of the dialysate circuit located inside the hemodialysis machine 102. As noted above, the fresh dialysate is circulated through various dialysate lines and dialysate components, including the dialyzer 110, that form the dialysate circuit. As will be described below, as the dialysate passes through the dialyzer 110, it collects toxins from the patient's blood. The resulting spent dialysate is carried from the dialysate circuit to a drain via the drain line 128. When ultrafiltration is performed during treatment, a combination of spent dialysate (described below) and excess fluid drawn from the patient is carried to the drain via the ultrafiltration line 129.

The dialyzer 110 serves as a filter for the patient's blood. The dialysate passes through the dialyzer 110 along with the blood, as described above. A semi-permeable structure (e.g., a semi-permeable membrane and/or semi-permeable microtubes) within the dialyzer 110 separates blood and dialysate passing through the dialyzer 110. This arrangement allows the dialysate to collect toxins from the patient's blood. The filtered blood exiting the dialyzer 110 is returned to the patient. The dialysate exiting the dialyzer 110 includes toxins removed from the blood and is commonly referred to as "spent dialysate." The spent dialysate is routed from the dialyzer 110 to a drain.

A drug pump 192 also extends from the front of the hemodialysis machine 102. The drug pump 192 is a syringe pump that includes a clamping mechanism configured to retain a syringe 178 of the blood component set 104. The drug pump 192 also includes a stepper motor configured to move the plunger of the syringe 178 along the axis of the syringe 178. A shaft of the stepper motor is secured to the plunger in a manner such that when the stepper motor is operated in a first direction, the shaft forces the plunger into the syringe, and when operated in a second direction, the shaft pulls the plunger out of the syringe 178. The drug pump 192 can thus be used to inject a liquid drug (e.g., heparin) from the syringe 178 into the blood circuit via a drug delivery line 174 during use, or to draw liquid from the blood circuit into the syringe 178 via the drug delivery line 174 during use.

The hemodialysis machine 102 includes a control unit 101 (e.g., a processor) configured to receive signals from and transmit signals to the display 118, the control panel 120, and a communication module 107 (e.g., a near field communication (NFC) transceiver, a sensor or a camera). The control unit 101 can also communicate with a server (e.g., an Internet server), another dialysis system, or another network resource. In various examples, communication with the server may be via wireless communication over a telecommunications network, may be via a wired Ethernet connection to the server, and/or may be via physical transfer of a computer readable medium between the server and the dialysis system 100, such as using a USB drive. The control unit 101 controls operating parameters of the hemodialysis machine 102 based at least in part on signals received by the display 118, the control panel 120, and the communication module 107.

The display 118 presents a user interface that may include vital signs of a patient, operational parameters of the dialysis treatment, and controls associated with the hemodialysis process. For example, the operational parameters may include ultrafiltration parameters, blood pump rate and information associated with the dialysate, hematocrit alert levels, blood pressure alarm limits, medicine infusion parameters, etc. The display 118 may include a touch screen through which the operator may interact and control the hemodialysis machine 102. For example, the operator may input various treatment parameters associated with the hemodialysis process.

The communication module 107 is configured to detect and communicate with the short-range wireless device--for example, an ID card, smart card, or smartphone 105 (which hereinafter may be generally referred to herein as an "ID card") of an operator or a patient-- when the device is within its wireless communication range. The communication between the communication module 107 and the ID card 105 is facilitated by a short-range wireless technology protocol, for example, a Bluetooth protocol or an RFID protocol, such as an NFC protocol. Because the ID card 105 is associated with an operator of the dialysis machine 102 or a patient, the communication module 107 detects information associated with the identity of the operator or patient. The communication module 107 communicates information associated with the operator or the patient to the control unit 101. In response, the control unit 101 may cause the hemodialysis machine 102 to perform an action, as described, for example, in U.S. Published Patent Application No. 2017/0168688. Similarly, when the ID card 105 is taken out of wireless communication range of the communication module 107 (e.g., the ID card 105 goes from being in wireless communication range of the communication module 107 to not being in wireless communication range of the communication module 107), the communication module 107 may send a signal to the control unit 101 indicating that the operator or patient is no longer present. In response, the control unit 101 may cause the hemodialysis machine 102 to perform an action.

The communication module 107 may also detect information associated with the identity of the operator or patient through a biometric authentication process that can include, for example, facial recognition, palm/fingerprint and iris recognition. The communication module may include a sensor (for example, a camera) that can take an image of the face, finger/palm or eye of the user, and use information associated with the image to identify the user. This may be achieved by comparing the information associated with the image with profile information in a database. As another alternative, the ID card 105 may be implemented as an app (or similar) on a device, such as a smartphone, tablet, etc. and the communication module 107 may detect presence of a particular operator or patient based on identification information from the app and/or identification information provided directly by the device (i.e., via an operating system of a smartphone).

The control unit 101 receives information associated with the identity of the operator or patient from the communication module 107 and compares the information with the interface configuration profiles for multiple users (operators and/or patients) stored in a database. The database may be stored on a storage device associated with the dialysis system 100 or located at an external storage device (for example a server or a storage device associated with a different dialysis system.) Based on the comparison, the control unit 101 may retrieve the interface configuration profile of the operator or patient. The specific user interface that appears on the display may be based on the retrieved interface configuration profile. For example, the operating parameters and the dialysis controls that constitute the user interface may be determined from the retrieved interface configuration profile. Additionally, identity of the operator or patient, and the identity of a supervisor of the operator may also be displayed.

The control unit 101 may communicate information associated with identity of the operator or patient and/or operational parameters of the dialysis machine 102 to a storage device or a mobile device. For example, the control unit 101 may communicate the identity of the operator or patient to a supervisor. The control unit 101 may identify the supervisor from an interface configuration profile associated with the operator. The communication, to the supervisor, may be in the form of a text message, email, social media type posting, or voicemail to a mobile device associated with the supervisor. Alternatively, or additionally, information associated with the identity of the operator or patient may be stored in a database. Storing or communicating the identity of the operator or patient and the operational parameter may allow for tracking of the dialysis process. For example, if an operator does not correctly perform the dialysis procedure, a supervisor may intervene. In another example, if an unauthorized user tries to use the system, the supervisor may undo the changes made by the unauthorized user.

The control unit 101 may be configured to add or modify an interface configuration profile. An operator or patient may register a short-range wireless device, for example the ID card 105, that is associated with the operator or patient, and input the desired interface configuration profile. This can be done through a touch screen on the display 118, the control panel 120 or a kiosk that is not a part of the dialysis system 100.

The control unit 101 may be configured to detect an alarm in the hemodialysis system 100, and based on the alarm, change the user interface that appears on the display 118. The alarm may be caused when the value of an operational parameter satisfies a criteria. For example, an alarm may occur when conductivity of the dialysate is above a certain value. This may require immediate attention from a nurse, and therefore the user interface that appears on the display 118 may be changed to a form expected by and/or familiar to the nurse. Although the control unit 101 is shown disposed in the dialysis machine 102, it is noted that in other embodiments, aspects of the control unit performed in connection with the maintenance notification techniques described herein may be performed by a processor or control unit located remotely from the dialysis machine 102. For example, the control unit 101 may transmit information corresponding to the maintenance notification techniques to a remote smartphone, such as represented by the ID card 105 for processing in connection with maintenance notification for the electromechanical components.

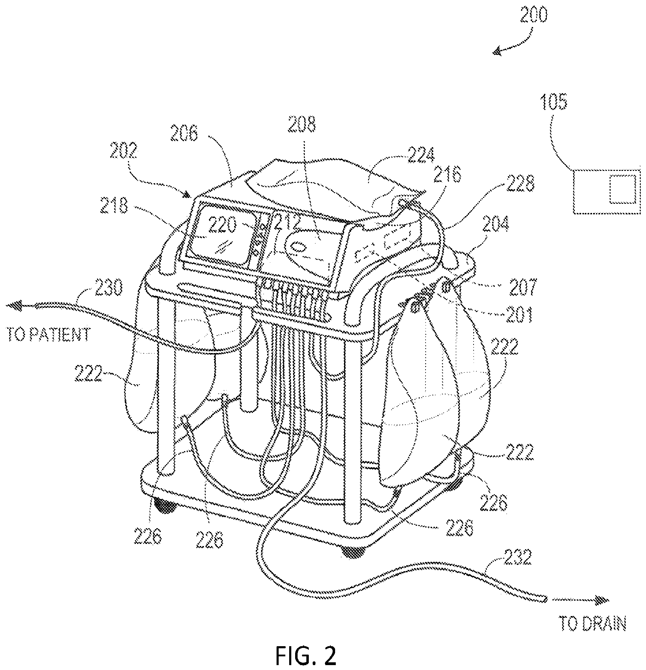

FIG. 2 shows a perspective view of a peritoneal dialysis (PD) system 200 that includes a PD cycler (also referred to as a PD machine) 202 seated on a cart 204. The PD cycler 202 includes a housing 206, a door 208, and a cassette interface (not shown) that contacts a disposable PD cassette 212 when the cassette 212 is disposed within a cassette compartment formed between the cassette interface and the closed door 208. A heater tray 216 is positioned on top of the housing 206. The heater tray 216 is sized and shaped to accommodate a bag of dialysate (e.g., a 5 liter bag of dialysate). The PD cycler 202 also includes a display 218 and additional control panel 220 that can be operated by a user (e.g., a patient) to allow, for example, set-up, initiation, and/or termination of a PD treatment.

Dialysate bags 222 are suspended from fingers on the sides of the cart 204, and a heater bag 224 is positioned in the heater tray 216. The dialysate bags 222 and the heater bag 224 are connected to the cassette 212 via dialysate bag lines 226 and a heater bag line 228, respectively. The dialysate bag lines 226 may be used to pass dialysate from dialysate bags 222 to the cassette 212 during use, and the heater bag line 228 may be used to pass dialysate back and forth between the cassette 212 and the heater bag 224 during use. In addition, a patient line 230 and a drain line 232 are connected to the cassette 212. The patient line 230 may be connected to a patient's abdomen via a catheter and may be used to pass dialysate back and forth between the cassette 212 and the patient's peritoneal cavity during use. The drain line 232 may be connected to a drain or drain receptacle and may be used to pass dialysate from the cassette 212 to the drain or drain receptacle during use. The various components that pass dialysate form a dialysate circuit.

The PD system 200, like the hemodialysis system 100, includes a control unit 201, a display 218 and a communication module 207 that communicate with one another, and with a short-range wireless device (for example, the ID card 105). Similar to the hemodialysis system 100, the PD system 200, using components such as the control unit 201 and the communication module 207, may detect information associated with the identity of an operator or patient, and change the user interface that appears on the display 218 based on an interface configuration profile. The PD system 200 allows an operator or patient to add or modify the interface configuration profile that may be stored in a storage device associated with the PD system 200, or an external storage device (for example, a server). Furthermore, the control unit 201 of the PD system 200 may change the user interface that appears on the display 218 based on an alarm in the PD system 200.

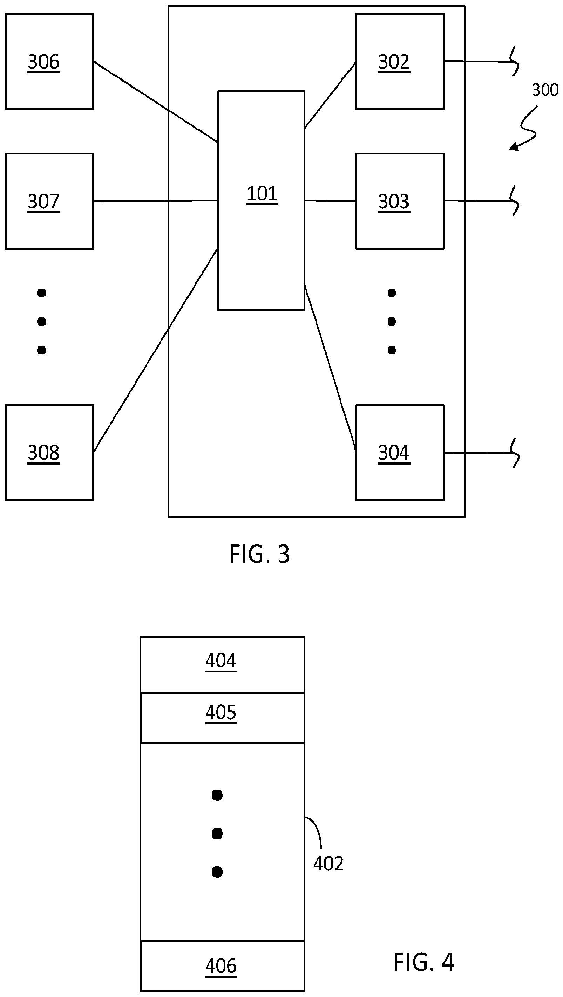

Referring to FIG. 3, a schematic diagram 300 shows the control unit 101 coupled to a plurality of actuators 302-304 and receiving signals from a plurality of sensors 306-308. The actuators 302-304 provide control signals to various electromechanical components of the hemodialysis system 100, such as the blood pump 132, the dialysate pump (not shown) used with the dialyzer 110, and the drug pump 192. Generally, the hemodialysis system 100 includes a number of different electromechanical components such as pumps, valves, actuators, sensors, gears, motors, solenoids, etc., each of which may be controlled by one of the actuators 302-304. Each of the actuators 302-304 may be adapted to provide appropriate signals to a specific electromechanical component that is being controlled thereby. For example, if the actuator 302 controls a particular pump that operates at a variable speed that is proportional to a voltage of between zero and nine volts, then the output of the actuator 302 may be a voltage signal that ranges from zero to nine volts. The actuator 302 may receive an input signal from the control unit 101 that is a digital command to the actuator to output a particular voltage signal within the predetermined range.

The sensors 306-308 receive information from different components of the hemodialysis system 100 and provide the information to the control unit 101. The information may be in the form of a digital signal that may be received and processed by the control unit 101. In many instances the sensors 306-308 are disposed proximal to whatever components are being measured by the sensors 306-308. Thus, for example, if the sensor 306 is a temperature sensor that measures temperature of the blood pump 132, the sensor 306 may be attached to or placed near the blood pump 132. As described in more detail elsewhere herein, in some instances the data that is being tabulated is not only a run count and/or run time of components, but also operational parameters such as temperature. This may be especially useful in instances where maintenance lifetime of a component is a function of both run time and operational conditions. For instance, a particular component may have a particular useful lifetime when operated at a particular temperature, but have a shorter lifetime when operated at a higher temperature.

Referring to FIG. 4, a table 402 includes a plurality of entries 404-406, each of which may correspond to a different component of the hemodialysis system 100. For example, the entry 404 may correspond to the blood pump 132. The table 402 may be maintained in non-volatile digital memory (e.g., a disk or an SSD memory) that is accessed by the control unit 101. Each of the entries 404-406 includes a first field that uniquely identifies a specific one of the components of the hemodialysis system 100 corresponding to the entry. Each of the entries 404-406 also includes a value corresponding to measured total run time, measured total number of cycles (i.e., number of times the corresponding component was actuated), or both. Each of the entries 404-406 may optionally include a secondary parameter, such as temperature, that is tracked along with run time/run cycle parameters. In some cases, the secondary parameter may be represented as an integration of the secondary parameter (i.e., the sum of measured values of the secondary parameter at each time measurement). It is also possible to store the secondary parameter as a count of the number of times the secondary parameter was measured to exceed a predetermined threshold. For example, the secondary parameter for one of the entries 404-406 may represent the number of times a temperature of a corresponding component was observed to be greater than a particular value. Of course, it is possible for an entry to have multiple secondary values corresponding to different counts for the same measurement exceeding different values (i.e., separate counts for a single temperature measurement exceeding a first threshold, a second threshold, etc.). It is also possible for an entry to have multiple secondary values corresponding to different measurements.

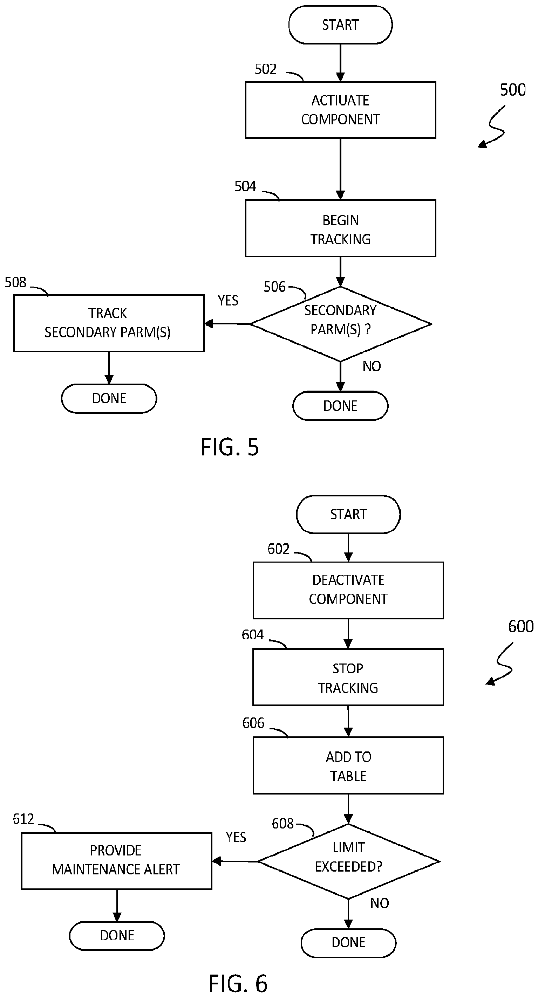

Referring to FIG. 5, a flow diagram 500 illustrates processing performed in connection with actuating a component of the hemodialysis system 100. Processing begins at a first step 502 where the component is actuated by, for example, the control unit 101 providing an appropriate command to an appropriate one of the actuators 302-304. Following the step 502 is a step 504 where a timer for the component is started. In an embodiment herein, the timer is used to keep track of how long the component has been actuated. In instances where only a count of a number of times a component is actuated is needed, the processing at the step 504 may set a Boolean variable to cause a counter to be incremented or may simply increment the counter directly. Following the step 504 is a test step 506 where it is determined if there are secondary parameters, such as temperature, associated with monitoring the component. If not, then processing is complete. Otherwise, control transfers from the test step 506 to a step 508 where the secondary parameter is monitored (e.g., a temperature reading is performed). Following the step 508, processing is complete.

Referring to FIG. 6, a flow diagram 600 illustrates processing performed in connection with deactivating a previously actuated component of the hemodialysis system 100. Processing begins at a first step 602 where the controller 101 provides a signal to an appropriate one of the actuators 302-304 to cause the previously actuated component to be deactivated. Following the step 602 is a step 604 where the tracking timer (set at the step 504, discussed above) is stopped. Of course, in instances where a tracking timer is not used (i.e., in instances where only a count of the actuation is tracked), there may be no timer to stop at the step 604. Following the step 604 is a step 606 where information from the timer (if used) and a possible secondary parameters (if used) are added to the table 402, discussed above. In the case of tracking total actuation time, the value from the timer (discussed above) may be added to a value of measured total run time for the component that is being deactivated.

Following the step 606 is a test step 608 where it is determined if any of the values for the entry exceed a predetermined limit that would cause the system to indicate that maintenance is due. For example, one of the entries 404-406 in the table 402 for the drug pump 192 may indicate that the drug pump 192 has been run for more time than a preset limit for the total amount of time that the drug pump 192 may run before needing maintenance. The predetermined limits may be independent from each other so that, for example, a run time limit for the drug pump 192 may be different and independent from a run time limit for the blood pump 132. The predetermined limits may be provided by a manufacturer of a device or may be based on empirical observations of similar systems. In some embodiments, it may be possible for the hemodialysis system 100 and other hemodialysis systems to provide maintenance information to a centralized database that analyzes prior maintenance information and determines predetermined limits that are used at the step 608 for different components. If it is determined at the step 608 that none of the values for the entry in the table 402 have not exceed any predetermined limits, then processing is complete. Otherwise, control transfers from the test step 608 to a step 612 where an alert is provided to indicate that the component requires maintenance. The alert at the step 612 may be provided in any appropriate fashion, including being displayed on the touch screen accessible display 118 or being communicated to a separate system (e.g., an email system) that provides notice to appropriate individuals for maintenance.

Note that even though the system described herein has been illustrated using the control unit 101 and the hemodialysis system 100, the system may be used for any type of medical equipment, including the peritoneal dialysis system 200 and the control unit 201. The system described herein may be adapted to any device or medical device in which different, independent, maintenance parameters are used for different components and where it is possible to detect and monitor actuation and deactivation of the components.

Although an example processing system has been described herein, implementations of the subject matter and the functional operations described above may be implemented in other types of digital electronic circuitry, or in computer software, firmware, or hardware, including the structures disclosed in this specification and their structural equivalents, or in combinations of one or more of them. Implementations of the subject matter described in this specification may be implemented as one or more computer program products, i.e., one or more modules of computer program instructions encoded on a tangible program carrier, for example a computer-readable medium, for execution by, or to control the operation of, a processing system. The computer readable medium may be a machine readable storage device, a machine readable storage substrate, a memory device, a composition of matter effecting a machine readable propagated signal, or a combination of one or more of them. A processing system may include, in addition to hardware, code that creates an execution environment for the computer program in question, e.g., code that constitutes processor firmware, a protocol stack, a database management system, an operating system, or a combination of one or more of them.

A computer program (also known as a program, software, software application, script, executable logic, or code) may be written in any form of programming language, including compiled or interpreted languages, or declarative or procedural languages, and it may be deployed in any form, including as a standalone 5 program or as a module, component, subroutine, or other unit suitable for use in a computing environment. A computer program does not necessarily correspond to a file in a file system. A program may be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program may be deployed to be executed on one computer or on multiple computers that are located at one site or distributed across multiple sites and interconnected by a communication network.

Computer readable media suitable for storing computer program instructions and data include all forms of non-volatile or volatile memory, media and memory devices, including by way of example semiconductor memory devices, e.g., EPROM, EEPROM, and flash memory devices; magnetic disks, e.g., internal hard disks or removable disks or magnetic tapes; magneto optical disks; and CD-ROM and DVD-ROM disks. The processor and the memory may be supplemented by, or incorporated in, special purpose logic circuitry. The components of the system may be interconnected by any form or medium of digital data communication, e.g., a communication network. Examples of communication networks include a local area network ("LAN") and a wide area network ("WAN"), e.g., the Internet.

A number of embodiments of the invention have been described. Nevertheless, it will be understood that various modifications may be made without departing from the scope of the invention. Accordingly, other embodiments are within the scope of the following claims.

## Claims

1. A medical device (100, 102, 200, 202), comprising:
a plurality of electromechanical components (132, 134, 178, 192, 302 to 304, 306 to 308);
a control unit (101, 201), coupled to the electromechanical components (132, 134, 178, 192, 302 to 304, 306 to 308), that actuates the electromechanical components (132, 134, 178, 192, 302 to 304, 306 to 308) and, for each of the electromechanical components (132, 134, 178, 192, 302 to 304, 306 to 308), maintains at least one of: a count of a number of times a respective one of the electromechanical components (132, 134, 178, 192, 302 to 304, 306 to 308) is actuated or a run time duration of the respective one of the electromechanical components (132, 134, 178, 192, 302 to 304, 306 to 308); and
a table (402) having observed values for each of the electromechanical components (132, 134, 178, 192, 302 to 304, 306 to 308), wherein a maintenance alert is provided for at least one of the electromechanical components (132, 134, 178, 192, 302 to 304, 306 to 308) in response to at least one of: the count or the run time for the at least one of the electromechanical components (132, 134, 178, 192, 302 to 304, 306 to 308) exceeding a corresponding maintenance limit for the electromechanical component (132, 134, 178, 192, 302 to 304, 306 to 308) and wherein the maintenance limits are independent for different ones of the electromechanical components (132, 134, 178, 192, 302 to 304, 306 to 308); and
a plurality of sensors (306 to 308), coupled to the control unit (101, 201), that measure a plurality of secondary parameters that cause the maintenance alert to be provided, wherein the table (402) maintains values for the secondary parameters;
**characterized in that** one of the secondary parameters is temperature.

2. A medical device (100, 102, 200, 202), according to claim 1, wherein the medical device is a dialysis machine.

3. A medical device (100, 102, 200, 202), according to claim 1, wherein at least some of the maintenance limits are determined by analyzing prior maintenance information.

4. A medical device (100, 102, 200, 202), according to claim 1, wherein the electromechanical components (132, 134, 178, 192, 302 to 304, 306 to 308) include at least one of: pumps, valves, actuators, sensors, gears, motors, or solenoids.

5. A method of determining when maintenance needs to be performed for electromechanical components (132, 134, 178, 192, 302 to 304, 306 to 308) of a medical device (100, 102, 200, 202), comprising:
maintaining in a table (402) at least one of: a count of a number of times a respective one of the electromechanical components (132, 134, 178, 192, 302 to 304, 306 to 308) is actuated or a run time duration of the respective one of the electromechanical components (132, 134, 178, 192, 302 to 304, 306 to 308) in connection with actuating each of the electromechanical components (132, 134, 178, 192, 302 to 304, 306 to 308); and
providing a maintenance alert for at least one of the electromechanical components (132, 134, 178, 192, 302 to 304, 306 to 308) in response to at least one of: the count or the run time for the at least one of the electromechanical components (132, 134, 178, 192, 302 to 304, 306 to 308) exceeding a corresponding maintenance limit for the electromechanical component, wherein the maintenance limits are independent for different ones of the electromechanical components (132, 134, 178, 192, 302 to 304, 306 to 308);
wherein the table (402) maintains values for secondary parameters that cause the maintenance alert to be provided, wherein a plurality of sensors (306 to 308), coupled to a control unit (101, 201), measures the secondary parameters,
**characterized in that** one of the secondary parameters is temperature.

6. A method, according to claim 5, wherein the medical device (100, 102, 200, 202) is a dialysis machine.

7. A method, according to claim 5, wherein at least some of the maintenance limits are determined by analyzing prior maintenance information.

8. A method, according to claim 5, wherein the electromechanical components (132, 134, 178, 192, 302 to 304, 306 to 308) include at least one of: pumps, valves, actuators, sensors, gears, motors, or solenoids.

9. A non-transitory computer readable medium containing software that determines when maintenance needs to be performed for electromechanical components (132, 134, 178, 192, 302 to 304, 306 to 308) of a medical device (100, 102, 200, 202), the software comprising:
executable code that maintains in a table (402) at least one of: a count of a number of times a respective one of the electromechanical components (132, 134, 178, 192, 302 to 304, 306 to 308) is actuated or a run time duration of the respective one of the electromechanical components (132, 134, 178, 192, 302 to 304, 306 to 308) in connection with actuating each of the electromechanical components (132, 134, 178, 192, 302 to 304, 306 to 308); and
executable code that provides a maintenance alert for at least one of the electromechanical components (132, 134, 178, 192, 302 to 304, 306 to 308) in response to at least one of: the count or the run time for the at least one of the electromechanical components (132, 134, 178, 192, 302 to 304, 306 to 308) exceeding a corresponding maintenance limit for the electromechanical component (132, 134, 178, 192, 302 to 304, 306 to 308), wherein the maintenance limits are independent for different ones of the electromechanical components (132, 134, 178, 192, 302 to 304, 306 to 308), wherein the table (402) maintains values for secondary parameters that cause the maintenance alert to be provided, wherein a plurality of sensors (306 to 308), coupled to a control unit (101, 201), measures the secondary parameters,
**characterized in that** one of the secondary parameters is temperature.

10. A non-transitory computer readable medium, according to claim 9, wherein the medical device (100, 102, 200, 202) is a dialysis machine.

11. A non-transitory computer readable medium, according to claim 9, wherein at least some of the maintenance limits are determined by analyzing prior maintenance information.

12. A non-transitory computer readable medium, according to claim 9, wherein the electromechanical components (132, 134, 178, 192, 302 to 304, 306 to 308) include at least one of: pumps, valves, actuators, sensors, gears, motors, or solenoids.

13. A dialysis system (100, 200), comprising:
a dialysis machine (102, 202), the dialysis machine (102, 202) including:
a housing (103, 206);
a dialysate circuit, at least a portion of which is disposed inside the housing (103, 206), the dialysate circuit circulating dialysate to remove toxins from blood of a patient coupled to the dialysis system (100, 200); and
a plurality of electromechanical components (132, 134, 178, 192, 302 to 304, 306 to 308); and
a control unit (101, 201) having a processor that executes software in a non-transitory computer readable medium, the software including executable code that maintains in a table (402) at least one of: a count of a number of times a respective one of the electromechanical components (132, 134, 178, 192, 302 to 304, 306 to 308) is actuated or a run time duration of the respective one of the electromechanical components (132, 134, 178, 192, 302 to 304, 306 to 308) in connection with actuating each of the electromechanical components (132, 134, 178, 192, 302 to 304, 306 to 308) and executable code that provides a maintenance alert for at least one of the electromechanical components (132, 134, 178, 192, 302 to 304, 306 to 308) in response to at least one of: the count or the run time for the at least one of the electromechanical components (132, 134, 178, 192, 302 to 304, 306 to 308) exceeding a corresponding maintenance limit for the electromechanical component (132, 134, 178, 192, 302 to 304, 306 to 308), wherein the maintenance limits are independent for different ones of the electromechanical components (132, 134, 178, 192, 302 to 304, 306 to 308), wherein the table (402) maintains values for secondary parameters that cause the maintenance alert to be provided, wherein a plurality of sensors (306 to 308), coupled to a control unit (101, 201), measures the secondary parameters,
**characterized in that** one of the secondary parameters is temperature.

## Patentansprüche

1. Medizinische Vorrichtung (100, 102, 200, 202), umfassend:
eine Mehrzahl elektromechanischer Komponenten (132, 134, 178, 192, 302 bis 304, 306 bis 308);
eine Steuereinheit (101, 201), welche mit den elektromechanischen Komponenten (132, 134, 178, 192, 302 bis 304, 306 bis 308) verbunden ist und welche die elektromechanischen Komponenten (132, 134, 178, 192, 302 bis 304, 306 bis 308) betätigt und für jede der elektromechanischen Komponenten (132, 134, 178, 192, 302 bis 304, 306 bis 308) mindestens eine der folgenden festhält: eine Zählung einer Anzahl Male, welche eine jeweilige elektromechanische Komponente (132, 134, 178, 192, 302 bis 304, 306 bis 308) betätigt wird, oder eine Laufzeitdauer der jeweiligen elektromechanischen Komponente (132, 134, 178, 192, 302 bis 304, 306 bis 308); und eine Tabelle (402), welche beobachtete Werte für jede der elektromechanischen Komponenten (132, 134, 178, 192, 302 bis 304, 306 bis 308) enthält, wobei ein Wartungsalarm für mindestens eine der elektromechanischen Komponenten (132, 134, 178, 192, 302 bis 304, 306 bis 308) gegeben wird als Reaktion darauf, dass mindestens eine der folgenden: die Zählung oder die Laufzeit für die mindestens eine der elektromechanischen Komponenten (132, 134, 178, 192, 302 bis 304, 306 bis 308) eine entsprechende Wartungsgrenze für die elektromechanische Komponente (132, 134, 178, 192, 302 bis 304, 306 bis 308) überschreitet, und wobei die Wartungsgrenzen für unterschiedliche elektromechanische Komponenten (132, 134, 178, 192, 302 bis 304, 306 bis 308) voneinander unabhängig sind; und
eine Mehrzahl Sensoren (306 bis 308), welche mit der Steuereinheit (101, 201) verbunden sind und welche eine Mehrzahl Sekundärparameter messen, die bewirken, dass der Wartungsalarm gegeben wird, wobei die Tabelle (402) Werte für die Sekundärparameter festhält;
**dadurch gekennzeichnet, dass** einer der Sekundärparameter die Temperatur ist.

2. Medizinische Vorrichtung (100, 102, 200, 202) nach Anspruch 1, wobei die medizinische Vorrichtung ein Dialysegerät ist.

3. Medizinische Vorrichtung (100, 102, 200, 202) nach Anspruch 1, wobei mindestens einige der Wartungsgrenzen durch Analysieren vorheriger Wartungsinformationen bestimmt werden.

4. Medizinische Vorrichtung (100, 102, 200, 202) nach Anspruch 1, wobei die elektromechanischen Komponenten (132, 134, 178, 192, 302 bis 304, 306 bis 308) mindestens eine der folgenden umfassen: Pumpen, Ventile, Betätigungselemente, Sensoren, Getriebe, Motoren oder Spulen.

5. Verfahren zum Bestimmen, wann Wartung für elektromechanische Komponenten (132, 134, 178, 192, 302 bis 304, 306 bis 308) einer medizinischen Vorrichtung (100, 102, 200, 202) durchgeführt werden soll, umfassend:
Festhalten in einer Tabelle (402) mindestens einer der folgenden: eine Zählung einer Anzahl Male, welche eine jeweilige elektromechanische Komponente (132, 134, 178, 192, 302 bis 304, 306 bis 308) betätigt wird, oder einer Laufzeitdauer der jeweiligen elektromechanischen Komponente (132, 134, 178, 192, 302 bis 304, 306 bis 308) in Verbindung mit dem Betätigen jeder der elektromechanischen Komponenten (132, 134, 178, 192, 302 bis 304, 306 bis 308); und
Geben eines Wartungsalarms für mindestens eine der elektromechanischen Komponenten (132, 134, 178, 192, 302 bis 304, 306 bis 308) als Reaktion darauf, dass mindestens eine der folgenden: die Zählung oder die Laufzeit für die mindestens eine der elektromechanischen Komponenten (132, 134, 178, 192, 302 bis 304, 306 bis 308) eine entsprechende Wartungsgrenze für die elektromechanische Komponente überschreitet, wobei die Wartungsgrenzen für unterschiedliche elektromechanische Komponenten (132, 134, 178, 192, 302 bis 304, 306 bis 308) voneinander unabhängig sind;
wobei die Tabelle (402) Werte für Sekundärparameter festhält, die bewirken, dass der Wartungsalarm gegeben wird, wobei eine Mehrzahl Sensoren (306 bis 308), welche mit einer Steuereinheit (101, 201) verbunden sind, die Sekundärparameter messen,
**dadurch gekennzeichnet, dass** einer der Sekundärparameter die Temperatur ist.

6. Verfahren nach Anspruch 5, wobei die medizinische Vorrichtung (100, 102, 200, 202) ein Dialysegerät ist.

7. Verfahren nach Anspruch 5, wobei mindestens einige der Wartungsgrenzen durch Analysieren vorheriger Wartungsinformationen bestimmt werden.

8. Verfahren nach Anspruch 5, wobei die elektromechanischen Komponenten (132, 134, 178, 192, 302 bis 304, 306 bis 308) mindestens eine der folgenden umfassen: Pumpen, Ventile, Betätigungselemente, Sensoren, Getriebe, Motoren oder Spulen.

9. Nichtflüchtiges computerlesbares Medium, welches Software enthält, die bestimmt, wann Wartung für elektromechanische Komponenten (132, 134, 178, 192, 302 bis 304, 306 bis 308) einer medizinischen Vorrichtung (100, 102, 200, 202) durchgeführt werden soll, wobei die Software Folgendes umfasst:
ausführbaren Code, welcher in einer Tabelle (402) mindestens eine der folgenden festhält: eine Zählung einer Anzahl Male, welche eine jeweilige elektromechanische Komponente (132, 134, 178, 192, 302 bis 304, 306 bis 308) betätigt wird, oder eine Laufzeitdauer der jeweiligen elektromechanischen Komponente (132, 134, 178, 192, 302 bis 304, 306 bis 308) in Verbindung mit dem Betätigen jeder der elektromechanischen Komponenten (132, 134, 178, 192, 302 bis 304, 306 bis 308); und
ausführbaren Code, welcher einen Wartungsalarm für mindestens eine der elektromechanischen Komponenten (132, 134, 178, 192, 302 bis 304, 306 bis 308) gibt als Reaktion darauf, dass mindestens eine der folgenden: die Zählung oder die Laufzeit für die mindestens eine der elektromechanischen Komponenten (132, 134, 178, 192, 302 bis 304, 306 bis 308) eine entsprechende Wartungsgrenze für die elektromechanische Komponente (132, 134, 178, 192, 302 bis 304, 306 bis 308) überschreitet, wobei die Wartungsgrenzen für unterschiedliche elektromechanische Komponenten (132, 134, 178, 192, 302 bis 304, 306 bis 308) voneinander unabhängig sind, wobei die Tabelle (402) Werte für Sekundärparameter festhält, die bewirken, dass der Wartungsalarm gegeben wird, wobei eine Mehrzahl Sensoren (306 bis 308), welche mit einer Steuereinheit (101, 201) verbunden sind, die Sekundärparameter messen,
**dadurch gekennzeichnet, dass** einer der Sekundärparameter die Temperatur ist.

10. Nichtflüchtiges computerlesbares Medium nach Anspruch 9, wobei die medizinische Vorrichtung (100, 102, 200, 202) ein Dialysegerät ist.

11. Nichtflüchtiges computerlesbares Medium nach Anspruch 9, wobei mindestens einige der Wartungsgrenzen durch Analysieren vorheriger Wartungsinformationen bestimmt werden.

12. Nichtflüchtiges computerlesbares Medium nach Anspruch 9, wobei die elektromechanischen Komponenten (132, 134, 178, 192, 302 bis 304, 306 bis 308) mindestens eine der folgenden umfassen: Pumpen, Ventile, Betätigungselemente, Sensoren, Getriebe, Motoren oder Spulen.

13. Dialysesystem (100, 200), umfassend:
ein Dialysegerät (102, 202), wobei das Dialysegerät (102, 202) Folgendes umfasst:
ein Gehäuse (103, 206);
einen Dialysatkreislauf, von welchem mindestens ein Teil innerhalb des Gehäuses (103, 206) angeordnet ist, wobei der Dialysatkreislauf Dialysat zirkulieren lässt, um Toxine aus Blut eines Patienten zu entfernen, welcher an das Dialysesystem (100, 200) angeschlossen ist; und
eine Mehrzahl elektromechanischer Komponenten (132, 134, 178, 192, 302 bis 304, 306 bis 308); und
eine Steuereinheit (101, 201) mit einem Prozessor, welcher Software in einem nichtflüchtigen computerlesbaren Medium ausführt, wobei die Software ausführbaren Code umfasst, welcher in einer Tabelle (402) mindestens eine der folgenden festhält: eine Zählung einer Anzahl Male, welche eine jeweilige elektromechanische Komponente (132, 134, 178, 192, 302 bis 304, 306 bis 308) betätigt wird, oder eine Laufzeitdauer der jeweiligen elektromechanischen Komponente (132, 134, 178, 192, 302 bis 304, 306 bis 308) in Verbindung mit dem Betätigen jeder der elektromechanischen Komponenten (132, 134, 178, 192, 302 bis 304, 306 bis 308), und ausführbaren Code, welcher einen Wartungsalarm für mindestens eine der elektromechanischen Komponenten (132, 134, 178, 192, 302 bis 304, 306 bis 308) gibt als Reaktion darauf, dass mindestens eine der folgenden: die Zählung oder die Laufzeit für die mindestens eine der elektromechanischen Komponenten (132, 134, 178, 192, 302 bis 304, 306 bis 308) eine entsprechende Wartungsgrenze für die elektromechanische Komponente (132, 134, 178, 192, 302 bis 304, 306 bis 308) überschreitet, wobei die Wartungsgrenzen für unterschiedliche elektromechanische Komponenten (132, 134, 178, 192, 302 bis 304, 306 bis 308) voneinander unabhängig sind, wobei die Tabelle (402) Werte für Sekundärparameter festhält, die bewirken, dass der Wartungsalarm gegeben wird, wobei eine Mehrzahl Sensoren (306 bis 308), welche mit einer Steuereinheit (101, 201) verbunden sind, die Sekundärparameter messen, **dadurch gekennzeichnet, dass** einer der Sekundärparameter die Temperatur ist.

## Revendications

1. Dispositif médical (100, 102, 200, 202) comprenant :
une pluralité de composants électromécaniques (132, 134, 178, 192, 302 à 304, 306 à 308) ;
une unité de commande (101, 201), couplée aux composants électromécaniques (132, 134, 178, 192, 302 à 304, 306 à 308), qui actionne les composants électromécaniques (132, 134, 178, 192, 302 à 304, 306 à 308) et, pour chacun des composants électromécaniques (132, 134, 178, 192, 302 à 304, 306 à 308), conserve au moins l'un des éléments suivants : un décompte d'un nombre de fois qu'un des composants électromécaniques (132, 134, 178, 192, 302 à 304, 306 à 308) est actionné ou une durée de fonctionnement de ce composant électromécanique (132, 134, 178, 192, 302 à 304, 306 à 308) ; et
un tableau (402) contenant des valeurs observées pour chacun des composants électromécaniques (132, 134, 178, 192, 302 à 304, 306 à 308), où une alerte de maintenance est fournie pour au moins un des composants électromécaniques (132, 134, 178, 192, 302 à 304, 306 à 308) en réponse à au moins l'un des éléments suivants : le décompte ou la durée de fonctionnement de l'au moins un des composants électromécaniques (132, 134, 178, 192, 302 à 304, 306 à 308) dépassant une limite de maintenance correspondante pour le composant électromécanique (132, 134, 178, 192, 302 à 304, 306 à 308) et où les limites de maintenance sont indépendantes pour différents composants électromécaniques (132, 134, 178, 192, 302 à 304, 306 à 308) ; et
une pluralité de capteurs (306 à 308), couplés à l'unité de commande (101, 201), qui mesurent une pluralité de paramètres secondaires qui provoquent l'alerte de maintenance, le tableau (402) conservant des valeurs des paramètres secondaires ;
**caractérisé en ce que** l'un des paramètres secondaires est la température.

2. Dispositif médical (100, 102, 200, 202) selon la revendication 1, le dispositif médical étant une machine de dialyse.

3. Dispositif médical (100, 102, 200, 202) selon la revendication 1, dans lequel au moins certaines des limites de maintenance sont déterminées par l'analyse d'informations de maintenance antérieures.

4. Dispositif médical (100, 102, 200, 202) selon la revendication 1, dans lequel les composants électromécaniques (132, 134, 178, 192, 302 à 304, 306 à 308) comprennent au moins l'un des éléments suivants : pompes, valves, actionneurs, capteurs, engrenages, moteurs ou solénoïdes.

5. Procédé pour déterminer quand une maintenance doit être effectuée pour des composants électromécaniques (132, 134, 178, 192, 302 à 304, 306 à 308) d'un dispositif médical (100, 102, 200, 202), comprenant les étapes suivantes :
conserver dans un tableau (402) au moins l'un des éléments suivants : un décompte d'un nombre de fois qu'un des composants électromécaniques (132, 134, 178, 192, 302 à 304, 306 à 308) est actionné ou une durée de fonctionnement de ce composant électromécanique (132, 134, 178, 192, 302 à 304, 306 à 308) en relation avec l'actionnement de chacun des composants électromécaniques (132, 134, 178, 192, 302 à 304, 306 à 308) ; et
fournir une alerte de maintenance pour au moins un des composants électromécaniques (132, 134, 178, 192, 302 à 304, 306 à 308) en réponse à au moins l'un des éléments suivants : le décompte ou la durée de fonctionnement de l'au moins un des composants électromécaniques (132, 134, 178, 192, 302 à 304, 306 à 308) dépassant une limite de maintenance correspondante pour le composant électromécanique, les limites de maintenance étant indépendantes pour différents composants électromécaniques (132, 134, 178, 192, 302 à 304, 306 à 308) ;
le tableau (402) conservant des valeurs de paramètres secondaires qui provoquent l'alerte de maintenance, une pluralité de capteurs (306 à 308), couplés à une unité de commande (101, 201), mesurant les paramètres secondaires,
**caractérisé en ce que** l'un des paramètres secondaires est la température.

6. Procédé selon la revendication 5, dans lequel le dispositif médical (100, 102, 200, 202) est une machine de dialyse.

7. Procédé selon la revendication 5, dans lequel au moins certaines des limites de maintenance sont déterminées par l'analyse d'informations de maintenance antérieures.

8. Procédé selon la revendication 5, dans lequel les composants électromécaniques (132, 134, 178, 192, 302 à 304, 306 à 308) comprennent au moins l'un des éléments suivants : pompes, vannes, actionneurs, capteurs, engrenages, moteurs ou solénoïdes.

9. Support non transitoire lisible par ordinateur contenant un logiciel qui détermine quand une maintenance doit être effectuée pour des composants électromécaniques (132, 134, 178, 192, 302 à 304, 306 à 308) d'un dispositif médical (100, 102, 200, 202), le logiciel comprenant :
un code exécutable qui conserve dans un tableau (402) au moins l'un des éléments suivants : un décompte d'un nombre de fois qu'un des composants électromécaniques (132, 134, 178, 192, 302 à 304, 306 à 308) est actionné ou une durée de fonctionnement de ce composant électromécanique (132, 134, 178, 192, 302 à 304, 306 à 308) en relation avec l'actionnement de chacun des composants électromécaniques (132, 134, 178, 192, 302 à 304, 306 à 308) ; et
un code exécutable qui fournit une alerte de maintenance pour au moins un des composants électromécaniques (132, 134, 178, 192, 302 à 304, 306 à 308) en réponse à au moins l'un des éléments suivants : le décompte ou la durée de fonctionnement de l'au moins un des composants électromécaniques (132, 134, 178, 192, 302 à 304, 306 à 308) dépassant une limite de maintenance correspondante pour le composant électromécanique (132, 134, 178, 192, 302 à 304, 306 à 308), les limites de maintenance étant indépendantes pour différents composants électromécaniques (132, 134, 178, 192, 302 à 304, 306 à 308), le tableau (402) conservant des valeurs de paramètres secondaires qui provoquent l'alerte de maintenance, une pluralité de capteurs (306 à 308), couplés à une unité de commande (101, 201), mesurant les paramètres secondaires,
**caractérisé en ce que** l'un des paramètres secondaires est la température.

10. Support non transitoire lisible par ordinateur selon la revendication 9, dans lequel le dispositif médical (100, 102, 200, 202) est une machine de dialyse.

11. Support non transitoire lisible par ordinateur selon la revendication 9, dans lequel au moins certaines des limites de maintenance sont déterminées par l'analyse d'informations de maintenance antérieures.

12. Support non transitoire lisible par ordinateur selon la revendication 9, dans lequel les composants électromécaniques (132, 134, 178, 192, 302 à 304, 306 à 308) comprennent au moins l'un des éléments suivants : pompes, vannes, actionneurs, capteurs, engrenages, moteurs ou solénoïdes.

13. Système de dialyse (100, 200) comprenant :
une machine de dialyse (102, 202), la machine de dialyse (102, 202) comprenant :
un boîtier (103, 206) ;
un circuit de dialysat dont au moins une partie est disposée à l'intérieur du boîtier (103, 206), le circuit de dialysat faisant circuler du dialysat pour éliminer des toxines du sang d'un patient relié au système de dialyse (100, 200) ; et
une pluralité de composants électromécaniques (132, 134, 178, 192, 302 à 304, 306 à 308) ; et
une unité de commande (101, 201) dotée d'un processeur qui exécute un logiciel sur un support non transitoire lisible par ordinateur, le logiciel comprenant un code exécutable qui conserve dans un tableau (402) au moins l'un des éléments suivants : un décompte d'un nombre de fois qu'un des composants électromécaniques (132, 134, 178, 192, 302 à 304, 306 à 308) est actionné ou une durée de fonctionnement de ce composant électromécanique (132, 134, 178, 192, 302 à 304, 306 à 308) en relation avec l'actionnement de chacun des composants électromécaniques (132, 134, 178, 192, 302 à 304, 306 à 308) et un code exécutable qui fournit une alerte de maintenance pour au moins un des composants électromécaniques (132, 134, 178, 192, 302 à 304, 306 à 308) en réponse à au moins l'un des éléments suivants : le décompte ou la durée de fonctionnement de l'au moins un des composants électromécaniques (132, 134, 178, 192, 302 à 304, 306 à 308) dépassant une limite de maintenance correspondante pour le composant électromécanique (132, 134, 178, 192, 302 à 304, 306 à 308), les limites de maintenance étant indépendantes pour différents composants électromécaniques (132, 134, 178, 192, 302 à 304, 306 à 308), le tableau (402) conservant des valeurs de paramètres secondaires qui provoquent l'alerte de maintenance, une pluralité de capteurs (306 à 308), couplés à une unité de commande (101, 201), mesurant les paramètres secondaires,
**caractérisé en ce que** l'un des paramètres secondaires est la température.
